# EUROPEAN PATENT SPECIFICATION

(11) **EP 4 310 461 B1**
(45) Date of publication and mention of the grant of the patent: **25.03.2026**
(21) Application number: 22185437.5
(22) Date of filing: 18.07.2022
(51) Int. Cl.: G01F 11/02, F04B 13/00, F04B 17/03, F04B 49/06, G01F 22/02, G01N 33/18, G01N 21/78

(54) **A PROCESS WATER ANALYZER**
PROZESSWASSERANALYSATOR
ANALYSEUR D'EAU DE PROCESSUS

(43) Date of publication of application: 24.01.2024
(73) Proprietor: Hach Lange GmbH, 14163 Berlin (DE)
(72) Inventor: GOLITZ, Andreas, 47475 Kamp-Lintfort (DE); BATTEFELD, Manfred, 40211 Düsseldorf (DE); DE HEIJ, Dr. Bas, 41542 Dormagen (DE); HEUWOLD, Maik, 47799 Krefeld (DE); MINKE, Sebastian, 47802 Krefeld (DE)
(74) Representative: terpatent PartGmbB

(56) References cited:
- US-A1- 2011 318 242
- US-A1- 2019 151 768

## Description

The invention is directed to a process water analyzer for automatically analyzing a parameter of a water sample.

A process water analyzer, as know from US 2019 0 151 768 A1, quasi-continuously determines a parameter of water. The parameter can be an analyte as, for example, the concentration of a substance, for example of ammonium or phosphate, or can be a more complex parameter as, for example, the total organic carbon (TOC). A process water analyzer comprises one or more process liquid reservoir tanks with process liquids which are necessary to provide the complete measurement process. Typical process liquids are reagents for providing a reaction with the analyte, for example a colour reaction, or are other process liquids, such as a cleaning liquid or a standard solution liquid.

For a precise dosage of the process liquid, a process water analyzer typically is provided with a very precise positive displacement dosage pump which allows to first suck the process liquid from a process liquid reservoir tank into a dosage chamber, and then to push a defined volume of the process liquid from the dosage chamber to an analyzer unit, for example to a photometer. A precise dosage arrangement is known from US 2011 0 318 242 A1.

A typical total volume of a process liquid reservoir tank is 1.5 to 5 I. Since the maintenance of a process water analyzer should be as simple as possible, a sufficiently precise determination of the process liquid reservoir volume in the corresponding reservoir tank is necessary to allow a precise forecast of the moment of exhaustion of the process liquid.

Typical tank liquid level sensors are based on mechanical, optical or acoustic principles, are not very precise, and cause relatively high costs because every single process liquid reservoir tank needs its own tank liquid level sensor.

It is an object of the invention to provide a process water analyzer with a simple and cost-effective means for determining the actual process liquid reservoir volume.

This object is solved with a process water analyzer with the features of main claim 1.

The process water analyzer according to the invention for automatically analyzing a parameter of a water sample comprises at least one process liquid reservoir tank with a process liquid, for example a colorimetric reagent, a cleaning liquid, a rinsing liquid and/or at least one standard solution liquid. The process liquid reservoir volume defines a reservoir liquid level at a reservoir liquid level height which is the vertical distance between the bottom of the reservoir tank and the level of the process liquid surface. The process liquid reservoir tank preferably is a disposable tank.

The process water analyzer is provided with a dosage chamber fluidically connected to the process liquid reservoir tank and being positioned fluidically downstream of the process liquid reservoir tank. The dosage chamber preferably is positioned vertically higher than the process liquid reservoir tank. The dosage chamber is a separate chamber which is used for precisely dosing the volume of the liquid sample and of the process liquids before the liquid is pumped to the analyzing unit.

The process water analyzer is provided with a positive displacement dosage pump fluidically connected to the top of the dosage chamber for sucking a defined process volume of the process liquid from the corresponding process liquid reservoir tank into the dosage chamber via a liquid line having an inlet opening in the bottom region of the process liquid reservoir tank and having the liquid line outlet opening at the bottom of the dosage chamber. An air cushion is always provided vertically between the dosage pump and the process liquid column upper level to avoid any direct contact of the dosage pump with the process liquid. This measure avoids any contamination of the dosage pump with any of the process liquids and thereby guarantees a long lifetime of the dosage pump.

If the process liquid reservoir tank is arranged vertically below the dosage chamber, a process liquid column with an effective vertical liquid column length is hanging below the air cushion and thereby expands the total volume of the air cushion so that the static gas pressure in the closed air cushion is reduced accordingly. The effective vertical liquid column length is the vertical distance between the liquid column upper level in the liquid line or in the dosage chamber and the reservoir liquid level in the reservoir tank. The reservoir liquid level has a reservoir level height above the reservoir tank bottom wall. The vertical height of the liquid column upper level is the vertical height with respect to the reservoir tank bottom wall of the process liquid reservoir tank, and is known sufficiently precisely at a particular moment.

The process water analyzer is provided with a gas pressure sensor fluidically connected to the dosage pump inlet/outlet opening for detecting and sensing the gas pressure of the air cushion provided above the liquid column upper surface. Preferably, the gas pressure sensor is fluidically provided in the top region of the dosage chamber or between the dosage chamber and the dosage pump.

The process water analyzer is provided with a liquid reservoir volume control unit calculating the actual process liquid reservoir volume based on the gas pressure of the air cushion provided by the gas pressure sensor, on the known vertical height of the liquid column upper level, on the known specific weight of the process liquid and on the known gas pressure of the air cushion right before a corresponding valve is opened to allow the process liquid column to expand the air cushion, the cushion expansion being caused by the weight of the effective process liquid column.

The vertical height of the liquid column upper level can be known or determined directly or indirectly in different ways and/or by different kinds of sensors. However, the actual vertical height of the liquid column upper level is known at least right before the gas pressure measurement is initiated by opening a corresponding valve so that the weight of the process liquid column is expanding the air cushion accordingly with the consequence that also the liquid column upper level is falling accordingly. The known vertical height of the liquid column upper level in context of the present invention preferably is the actual vertical height of the liquid column upper level at the moment before the weight of the process liquid column expands the air cushion.

The pressure difference between the gas pressure measured by the pressure sensor and the environmental atmospheric pressure is approximately proportional to the vertical liquid column length of the effective liquid column, whereas the upper column liquid column end is defined by the liquid column upper level and the lower liquid column end is defined by the reservoir liquid level of the process liquid in the fluidically connected process liquid reservoir tank. Since the specific weight of the corresponding process liquid is known, the total vertical extent of the effective liquid column can be determined straight forward.

The correlation between the process liquid level height in the reservoir tank and the process liquid reservoir volume is known, for example is memorized in a lookup table, so that the actual process liquid reservoir volume can simply be concluded and/or calculated on the basis of the effective vertical liquid column length determined on the basis of the gas pressure sensed by the gas pressure sensor.

No tank liquid level sensors are necessary anymore to determine the actual process liquid reservoir volume in any of the process liquid reservoir tanks so that a simple, sufficiently precise and inexpensive means is provided for determining the actual process liquid reservoir volumes of all process liquid reservoir tanks.

Preferably, the dosage chamber is provided with a liquid outlet opening and a corresponding outlet valve, which together allow to set the process liquid column to a defined vertical height of the liquid column upper level which is the vertical height position of the corresponding liquid outlet opening of the dosage chamber. The liquid outlet opening of the dosage chamber can have different primary functions, and preferably is a part of liquid line connecting of the dosage chamber with an analyzer unit.

After a new and completely filled process liquid reservoir tank has been applied to the process water analyzer, and if the reservoir liquid level of the completely filled process liquid reservoir tank should not be known precisely, a reference measurement can be provided. The dosage pump sucks the process liquid to the dosage chamber so that the process liquid in the dosage chamber finally is at least minimally above the liquid outlet opening. The dosage pump is stopped, a tank valve provided in the fluid line between the reservoir tank and the dosage chamber is closed, and the process liquid volume above the reference outlet opening flows or is pumped out of the dosage chamber through the liquid outlet opening.

Finally, the liquid column upper level in the dosage chamber remains at the vertical position of the liquid outlet opening of which the vertical hight position with respect to the bottom wall of the process liquid reservoir tank is known.

The tank valve is opened so that the weight of the process liquid column now expands the air cushion so that the pressure of the air cushion decreases accordingly. The gas pressure is sensed by the gas pressure sensor, and the liquid reservoir volume control unit determines the individual process liquid reservoir volume of the corresponding process liquid reservoir tank.

After that, a continuous control of the process liquid reservoir volume by means of gas pressure measurements is not necessary anymore, because a sufficiently precise calculation of the consumption of the corresponding process liquid can be based on a recording and an interpretation of the pump activities.

Alternatively, the liquid reservoir volume control unit calculates the actual process liquid reservoir volume in constant intervals or even during every dosing of the process liquid. The process water analyzer according to the invention allows to indirectly determine the actual process liquid reservoir volume always when the corresponding process liquid is consumed in the analyzing process.

The dosage chamber alternatively or additionally can be provided with a simple one-point liquid level sensor which alternatively or additionally to a liquid outlet opening allows to define a known vertical height of the liquid column upper level in the dosage chamber. The one-point liquid level sensor can be, for example, an optical sensor.

Preferably, the dosage pump is a piston pump. The piston pump pumps the fluids very precisely and with a high constancy.

Preferably, the dosage pump is provided with an electric step motor. The electric step motor in the context of the present invention is every motor which allows to precisely control and record the number of motor rotor rotation steps. This allows to calculate the consumption of the process liquid on the basis of the recorded motor rotor steps for sucking the corresponding process liquid into the dosage chamber, from where the process liquid is induced into the measurement process.

Preferably, the gas pressure sensor is a differential gas pressure sensor measuring the pressure difference of the gas pressure in relation to the atmospheric pressure of the direct process water analyzer environment. The differential gas pressure sensor compensates effects resulting from any deviation of the actual environmental atmospheric pressure from a standard pressure of, for example, 1013 mbar. Using a differential gas pressure sensor therefore makes a precise determination of the actual atmospheric pressure of the water analyzer environment redundant.

Preferably, a total atmospheric pressure sensor can be provided to determine the total atmospheric pressure as a reference.

The liquid reservoir volume control unit can optionally and additionally be provided with a pumping recorder quantitatively recording all pumping actions after a reference determination of the process liquid reservoir volume of the, for example, completely filled new process liquid reservoir tank. The pumping recorder allows to calculate the ongoing consumption of the corresponding process liquid because the volumetric pump performance is roughly proportional to the I see one the consumption of the pumped process liquid. This approach allows to reduce the gas-pressure-based determination of the process liquid reservoir volume to an interval of, for example once per day.

One embodiment of the invention is explained with reference to the enclosed drawing.

The figure schematically shows a process water analyzer according to the invention with a liquid reservoir volume control unit using a gas pressure sensor for the determination of the remaining process liquid volume in the corresponding process liquid reservoir tanks.

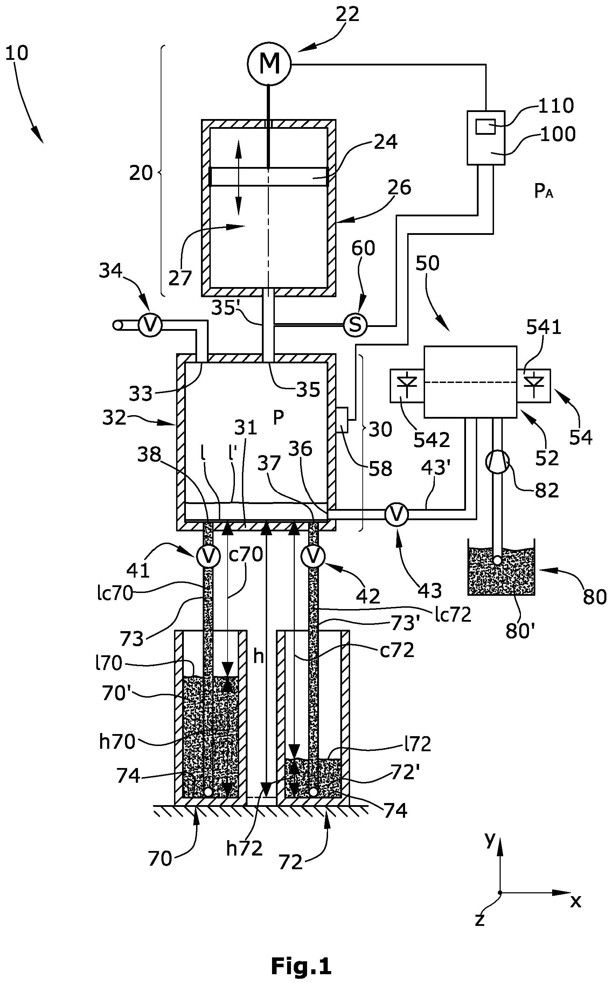

The figure 1 schematically shows a process water analyzer 10 for automatically analyzing a parameter of a water sample coming from a wastewater plant basin 80 filled with wastewater 80'. The analyzer 10 is provided with a photometer 50 photometrically analyzing and determining the concentration of a parameter of the water sample pumped by a sample pump 82 from the wastewater basin 80 to a photometric measurement chamber 52 of the photometer 50. The photometric measurement chamber 52 is provided with a photometric unit 54 comprising a photometric light source 541 and a photometric receiver 542.

In the figure, the vertical direction is y, and the horizontal plane is defined by x and z.

The process water analyzer 10 also comprises two process liquid reservoir tanks 70, 72 with process liquids 70', 72', a dosage chamber 30 fluidically connected to the process liquid reservoir tanks 70, 72 by a vertical supply line 73, 73', respectively, and a positive displacement dosage pump 20. However, six or even more different process liquid reservoir tanks could be provided.

The process liquids 70', 72' are, in the present embodiment, a reagent liquid and a cleaning liquid. The reagent liquid can be a reagent for the colorimetric determination of, for example, phosphate. The actual process liquid reservoir volumes V70 and V72 in the corresponding reservoir tank 70,72 result in a reservoir liquid level I70, I72 having a vertical reservoir level hight h70, h72 above the bottom wall surface 74 of the corresponding reservoir tanks 70, 72. A typical total liquid volume of a new and completely filled process liquid reservoir tank is of, for example, 2.0 liter.

The dosage chamber 30 having a typical volume of 3.6 ml is located vertically higher than the process liquid reservoir tanks 70, 72 and is fluidically connected to the process liquid reservoir tanks 70, 72 via two process liquid inlet openings 37,38 at the horizontal bottom wall 31 of the dosage chamber housing 32. The process liquid inlet openings 37, 38 define the vertical top end of the supply lines 73, 73', respectively. The vertical downward end of the supply lines 73, 73' is provided close to the bottom wall surface 74 of the corresponding reservoir tank 70, 72. A switchable supply line valve 41, 42 is provided in the course of every supply line 73, 73'.

At the top wall of the dosage chamber 30, a venting opening 33 is provided which allows a venting of the dosage chamber interior if a switchable venting valve 34 is open. A pump line opening 35 is provided at the top wall of the dosage chamber 30 and at the highest position of the dosage chamber 30. The interior of the dosage chamber 30 is fluidically connected to the dosage pump 20 via the pump line opening 35 at the top of the dosage chamber 30 and a pump line 35'. A gas pressure sensor 60 is provided at the pump line 35'. The dosage chamber 30 is provided with an optical one-point liquid level sensor 58 which detects the absence or the presence of a liquid at the vertical level of the liquid level sensor 58.

The dosage chamber 30 is provided with an analyzer liquid outlet opening 36 fluidically connecting the dosage chamber interior via a connection line 43' and a switchable photometer line valve 43 with the photometer measurement chamber 52. After the corresponding photometric measurement has been provided, the liquid of the photometer measurement chamber 52 is drained into a waste liquid container.

The positive displacement dosage pump 20 is provided with an electric pump motor 22 which is an electric step motor and which actuates a pump piston 24 linearly moving within a pump cylinder 26. The total displacement of the dosage pump 20 is 5.0 ml and the total number of motor steps for actuating the piston 24 from one pump cylinder end to the other pump cylinder end is 5000 steps.

An air cushion 27 is provided and located fluidically between the pump piston 24 of the dosage pump 20 and the actual liquid column upper level I of the liquid in the dosage chamber 30. The gas pressure sensor 60 is a differential sensor measuring the pressure difference between the gas pressure P of the air cushion 27 and the atmospheric pressure PA of the environment of the analyzer 10.

The process water analyzer 10 comprises a liquid reservoir volume control unit 100 which is provided with a pumping recorder 110 recording all pumping actions and motor steps of the dosage pump 20. The control unit 100 receives the pressure signal of the gas pressure sensor 60 and a liquid presence indication signal of the liquid level sensor 58. The control unit 100 also controls the photometer 50, the sample pump 22, and all switchable valves 34,41,42,43.

After new process liquid reservoir tanks 70, 72 have been applied to the water process water analyzer 10, an initial automatic reference measurement is provided to verify if the new process liquid reservoir tank 70, 72 are completely filled and are filled with the correct process liquid. This initial reference measurement is controlled by the control unit 100:

For the reference measurement of the first process liquid reservoir tank 70, the first supply line valve 41 is opened, and the second supply line valve 42, the photometer line valve 43 and the venting valve 34 are closed. The dosage pump 20 sucks a predefined priming volume Vprime of the first process liquid 70' through the corresponding supply line 73 into the dosage chamber 30. The value of the priming volume Vprime is sufficiently large to guarantee that the liquid column upper level I' is definitely above the liquid outlet opening 36, as shown in figure 1.

Then, the first supply line valve 41 is closed, the second supply line valve 42 remains closed and the photometer line valve 43 is opened, so that the process liquid in the dosage chamber 30 is pumped by the activated dosage pump 20 through the liquid outlet opening 36 to the photometer 50. The liquid in the dosage chamber 30 falls to a liquid column upper level I where the liquid level remains. This liquid column upper level I preferably is precisely in the horizontal plane defined by the liquid inlet openings 37, 38.

The total vertical height h of the liquid column upper level I with respect to the bottom wall surface 74 of the process liquid reservoir tank 70, 72 is constant and is a known value, and is memorized in the liquid reservoir volume control unit 100.

In the next process step, the photometer line valve 43 is closed and the venting valve 34 is shortly opened and then closed again to have the atmospheric pressure of the environment as a defined reference dosage chamber pressure P within the dosage chamber 30. The first supply line valve 41 now is opened, so that the weight of the vertical liquid column Ic70 between the liquid column upper level I and the reservoir liquid level I70 expands the air cushion 27 so that the air cushion 27 has now a total gas pressure P below the environmental atmospheric pressure PA.

The specific weight of the first process liquid 70' is known and is memorized in the control unit 100 so that the control unit 100 can determine or calculate from the pressure P the total vertical liquid column length c70, determines from the liquid column length c70 the reservoir liquid level I70, and finally determines from the reservoir liquid level I70 the actual process liquid reservoir volume V70 of the process liquid reservoir tank 70. The control unit 100 now can verify if the determined liquid reservoir volume V70 is plausible for a new and completely filled process liquid reservoir tank 70.

The same procedure is applied accordingly for the other new liquid reservoir tank 72, so that the control unit 100 determines the liquid column length c72 and the resulting liquid process liquid reservoir volume v72 of the second liquid reservoir tank 72, and finally controls the plausibility of the determined liquid reservoir volume V72.

The control unit 100 makes a first very rough prognosis of the moment of exhaustion of each of the liquid reservoir tanks 70, 72.

The pumping recorder 110 of the reservoir volume control unit 100 records all dosage pumping actions and pump motor steps of the dosage pump 20 sucking one of the process liquids 70', 72' from the process liquid reservoir tanks 70, 72 into the dosage chamber 30, and continuously sums up the consumed volumes of the process liquid 70', 72' so that the remaining actual process liquid reservoir volumes V70, V72 and their exhaustion can always be roughly calculated.

From time to time, in short intervals or during every process liquid dosage process step, the control unit 100 can provide the described determination of the corresponding process liquid reservoir volume V70, V72.

## Claims

1. A process water analyzer (10) for automatically analyzing a parameter of a water sample, comprising
a process liquid reservoir tank (70,72) with a process liquid (70',72'), whereas the process liquid reservoir volume (V70,V72) in the reservoir tank (70,72) defines a reservoir liquid level (I70,I72) at a reservoir liquid level height (h70, h72),
a dosage chamber (30) fluidically connected to the process liquid reservoir tank (70,72) and being positioned fluidically downstream of the process liquid reservoir tank (70,72),
a positive displacement dosage pump (20) fluidically connected to the top of the dosage chamber (30) for sucking the process liquid (70', 72') into the dosage chamber (30),
whereas an air cushion (27) is always provided vertically between the dosage pump (20) and a liquid column upper level (I) of a process liquid column (Ic70,Ic72) with an effective vertical liquid column length (c70,c72) between the reservoir liquid level (I70,I72) and the liquid column upper level (I),
a gas pressure sensor (60) sensing the gas pressure (P) of the air cushion (27), and
a liquid reservoir volume control unit (100) calculating the actual process liquid reservoir volume (V70,V72) on the basis of the gas pressure (P) provided by the gas pressure sensor (60) and the known vertical height (h) of the liquid column upper level (I).

2. The process water analyzer (10) of claim 1, wherein the dosage chamber (30) is provided with a liquid outlet opening (36) and a corresponding liquid outlet valve (43) which allow to set the liquid column upper level (I) of the process liquid column (Ic70,Ic72) to the defined vertical height (h) of the liquid outlet opening (36) of the dosage chamber (30).

3. The process water analyzer (10) of one of the preceding claims, wherein the liquid reservoir tank (70, 72) is provided free of a liquid level sensor.

4. The process water analyzer (10) of claim 1 or 2, wherein the dosage pump (20) is a piston pump.

5. The process water analyzer (10) of one of the preceding claims, wherein the dosage pump (20) is provided with an electric step motor (22).

6. The process water analyzer (10) of one of the preceding claims, wherein the gas pressure sensor (60) is a differential sensor measuring the pressure difference of the gas pressure (P) to the atmospheric pressure (PA).

## Patentansprüche

1. Ein Prozess-Wasseranalysator (10) zum automatischen Analysieren eines Parameters einer Wasserprobe, umfassend
einen Prozessflüssigkeits-Vorratstank (70, 72) mit einer Prozessflüssigkeit (70', 72'), wobei das Prozessflüssigkeits-Vorratsvolumen (V70, V72) in dem Vorratstank (70, 72) einen Vorratsflüssigkeits-Pegel (I70, I72) bei einer Vorratsflüssigkeits-Pegelhöhe (h70, h72) definiert,
eine Dosierkammer (30), die fluidisch mit dem Prozessflüssigkeits-Vorratstank (70, 72) verbunden ist und fluidisch stromabwärts von dem Prozessflüssigkeits-Vorratstank (70, 72) angeordnet ist,
eine Verdränger-Dosierpumpe (20), die fluidisch mit der Oberseite der Dosierkammer (30) verbunden ist, um die Prozessflüssigkeit (70', 72') in die Dosierkammer (30) zu saugen,
wobei ein Luftpolster (27) immer vertikal zwischen der Dosierpumpe (20) und einem oberen Flüssigkeitssäulen-Pegel (I) einer Prozessflüssigkeitssäule (Ic70, Ic72) mit einer effektiven vertikalen Flüssigkeitssäulen-Länge (c70, c72) zwischen dem Vorratsflüssigkeits-Pegel (I70, I72) und dem oberen Flüssigkeitssäulen-Pegel (I) vorgesehen ist,
einen Gasdrucksensor (60), der den Gasdruck (P) des Luftpolsters (27) erfasst, und
eine Flüssigkeitsvorratsvolumen-Steuereinheit (100), die das tatsächliche Prozessflüssigkeits-Vorratsvolumen (V70, V72) auf der Basis des von dem Gasdrucksensor (60) bereitgestellten Gasdrucks (P) und der bekannten vertikalen Höhe (h) des oberen Flüssigkeitssäulen-Pegels (I) berechnet.

2. Der Prozess-Wasseranalysator (10) nach Anspruch 1, wobei die Dosierkammer (30) mit einer Flüssigkeitsauslass-Öffnung (36) und einem entsprechenden Flüssigkeitsauslass-Ventil (43) versehen ist, die es ermöglichen, den oberen Flüssigkeitssäulen-Pegel (I) der Prozessflüssigkeits-Säule (Ic70, Ic72) auf die definierte vertikale Höhe (h) der Flüssigkeitsauslass-Öffnung (36) der Dosierkammer (30) einzustellen.

3. Der Prozess-Wasseranalysator (10) nach einem der vorhergehenden Ansprüche, wobei der Flüssigkeitsvorratstank (70, 72) frei von einem Flüssigkeitspegel-Sensor bereitgestellt ist.

4. Der Prozess-Wasseranalysator (10) nach Anspruch 1 oder 2, wobei die Dosierpumpe (20) eine Kolbenpumpe ist.

5. Der Prozess-Wasseranalysator (10) nach einem der vorhergehenden Ansprüche, wobei die Dosierpumpe (20) mit einem elektrischen Schrittmotor (22) ausgestattet ist.

6. Der Prozess-Wasseranalysator (10) nach einem der vorhergehenden Ansprüche, wobei der Gasdrucksensor (60) ein Differentialsensor ist, der die Druckdifferenz des Gasdrucks (P) zum atmosphärischen Druck (PA) misst.

## Revendications

1. Un analyseur de processus d'eau (10) pour l'analyse automatique d'un paramètre d'un échantillon d'eau, comprenant
un réservoir de liquide de processus (70, 72) avec un liquide de processus (70', 72'), tandis que le volume de réservoir de liquide de processus (V70, V72) dans le réservoir (70, 72) définit un niveau de liquide de réservoir (I70, I72) à une hauteur de niveau de liquide de réservoir (h70, h72),
une chambre de dosage (30) reliée fluidiquement au réservoir de liquide de processus (70, 72) et étant positionnée fluidiquement en aval du réservoir de liquide de processus (70, 72),
une pompe de dosage volumétrique (20) reliée fluidiquement à la partie supérieure de la chambre de dosage (30) pour aspirer le liquide de processus (70', 72') dans la chambre de dosage (30),
tandis qu'un coussin d'air (27) est toujours prévu verticalement entre la pompe de dosage (20) et un niveau supérieur de colonne de liquide (I) d'une colonne de liquide de processus (Ic70, Ic72) avec une longueur de colonne de liquide verticale effective (c70, c72) entre le niveau de liquide de réservoir (I70, I72) et le niveau supérieur de colonne de liquide (I),
un capteur de pression de gaz (60) détectant la pression de gaz (P) du coussin d'air (27), et
une unité de controle de volume de réservoir de liquide (100) calculant le volume de réservoir de liquide de processus réel (V70, V72) sur la base de la pression de gaz (P) fournie par le capteur de pression de gaz (60) et de la hauteur verticale connue (h) du niveau supérieur de colonne de liquide (I).

2. L'analyseur de processus d'eau (10) selon la revendication 1, dans lequel la chambre de dosage (30) est pourvue d'une ouverture de sortie de liquide (36) et d'une soupape de sortie de liquide correspondante (43) qui permettent de régler le niveau supérieur de colonne de liquide (I) de la colonne de liquide de processus (Ic70, Ic72) à la hauteur verticale définie (h) de l'ouverture de sortie de liquide (36) de la chambre de dosage (30).

3. L'analyseur de processus d'eau (10) selon l'une des revendications précédentes, dans lequel le réservoir de liquide (70, 72) est fourni sans capteur de niveau de liquide.

4. L'analyseur de processus d'eau (10) selon la revendication 1 ou 2, dans lequel la pompe de dosage (20) est une pompe à piston.

5. L'analyseur de processus d'eau (10) selon l'une des revendications précédentes, dans lequel la pompe de dosage (20) est fournie avec un moteur électrique pas à pas (22).

6. L'analyseur de processus d'eau (10) selon l'une des revendications précédentes, dans lequel le capteur de pression de gaz (60) est un capteur différentiel mesurant la différence de pression de la pression de gaz (P) à la pression atmosphérique (PA).
